Europäisches Patentamt

European Patent Office

Office européen des brevets

(11) Veröffentlichungsnummer: **0 144 017**
**B1**

(12) **EUROPÄISCHE PATENTSCHRIFT**

(45) Veröffentlichungstag der Patentschrift:
**27.01.88**

(21) Anmeldenummer: **84113701.1**

(22) Anmeldetag: **14.11.84**

�================

(51) Int. Cl.⁴: **C 12 P 7/62** // (C12P7/62,
C12R1:05)

(54) Verfahren zur biotechnologischen Herstellung von Poly-D(-)-3-hydroxybuttersäure.

(30) Priorität: **01.12.83 DE 3343551**

(43) Veröffentlichungstag der Anmeldung:
**12.06.85 Patentblatt 85/24**

(45) Bekanntmachung des Hinweises auf die Patenterteilung:
**27.01.88 Patentblatt 88/4**

(84) Benannte Vertragsstaaten:
**AT BE CH DE FR GB IT LI LU NL SE**

(56) Entgegenhaltungen:
**EP - A - 0 046 344**

**CHEMICAL ABSTRACTS, Band 95, Nr. 23, 3. Dezember 1981, Seite 341, Nr. 210413, COLUMBUS OHIO (US), K.A. MALIK et al.: "Nitrogen fixation by the hydrogen-oxidizing bacterium Alcaligenes latus"
CHEMICAL ABSTRACTS, Band 91, Nr. 3, 20. Juli 1979, Seite 307, Nr. 189420e, COLUMBUS OHIO (US), D. VOLLBRECHT et al.: "Excretion of metabolites by hydrogen bacteria. IV. Respiration ratedependent formation of primary metabolites and of poly-r-hydroxybutanoate"**

(73) Patentinhaber: **CHEMIE LINZ AKTIENGESELLSCHAFT, St. Peter-Strasse 25, A-4020 Linz (AT)**

(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Lafferty, Robert M. Dr., Dolezalgasse 21, A-8051 Graz (AT)**
Erfinder: **Braunegg, Gerhart, Dr. Dipl.-Ing., Kurzeggerweg 14, A-8044 Graz (AT)**

## Beschreibung

Die vorliegende Erfindung betrifft ein Verfahren zur biotechnologischen Herstellung von Poly-D(−)-3--hydroxybuttersäure mit einem hohen Ertragskoeffizienten und einer verbesserten Anreicherung von PHB im bakteriellen Zellmaterial, bei dem ein Mikroorganismus in zwei getrennten, aufeinanderfolgenden Fermentierungsstufen kontinuierlich gezüchtet wird.

Es ist seit längerer Zeit bekannt, dass eine Vielzahl von Mikroorganismen prokaryotischer Natur imstande ist, PHB als Speicherstoff für Energie und Kohlenstoff zellintern anzuhäufen. Die aus dem Zellmaterial des Mikroorganismus isolierte PHB ist ein thermoplastischer Polyester mit günstigen physikalischen Eigenschaften, die seine Verwendung für ähnliche Zwecke erwarten lassen, für welche heute beispielsweise Polyäthylen oder Polystyrol zur Verfügung stehen. Gegenüber diesen heute gebräuchlichen Polymeren hat die PHB aber den Vorteil, dass sie auf biotechnologischem Weg zugänglich und auf biologischem Weg auch wieder abbaubar ist.

Es ist auch schon bekannt, dass die aerobe Kultivierung von PHB-speichernden Mikroorganismen durch die spezifische Zusammensetzung des Nährmediums bevorzugt in Richtung von Zellteilung und Wachstum oder in Richtung von zellinterner PHB-Speicherung gesteuert werden kann. Eine Anreicherung von PHB in den Zellen des Mikroorganismus wird bei den bekannten Verfahren dann erreicht, wenn im Nährmedium die Konzentration der Kohlenstoffquelle im Verhältnis zum Angebot an anderen für das Wachstum erforderlichen Nährstoffen, beispielsweise Stickstoff und Phosphor, gross ist und die Züchtung des Mikroorganismus beispielsweise unter ammoniumlimitierten Wachstumsbedingungen erfolgt.

Ein auf diesen Erkenntnissen beruhendes zweistufiges Verfahren zur Herstellung von PHB ist z.B. in der EP-A-15 669 beschrieben. Bei diesem Verfahren wird ein Mikroorganismus von der Gattung Methylobacterium organophilium zunächst bei unbeschränkter Versorgung mit Nährstoffen, einschliesslich einer vollständigen und ausreichenden Versorgung mit Stickstoff und Phosphor, gezüchtet, bis die Mikroorganismenpopulation eine Konzentration von vorzugsweise 20 bis 25 g Biomasse pro Liter Kulturflüssigkeit erreicht hat, ohne dass in dieser Wachstumsphase eine zellinterne PHB-Speicherung auftritt. Die vollständige Unterbrechung der Stickstoff- und/oder Phosphorversorgung des Kulturmediums in der zweiten Fermentationsstufe bewirkt dann einen Stillstand der Reproduktion und des Bakterienwachstums, wobei erst in dieser Phase zellintern PHB-Speicherung eintritt. Nach den Angaben in der EP-A-15 669 werden bei diesem Verfahren Biomassen mit einem PHB-Gehalt von höchstens 25 bis 47 Gew.-% des Zelltrockengewichts erhalten.

In der EP-A-46 344 ist ein demgegenüber verbessertes Fermentationsverfahren für die Herstellung von PHB beschrieben, welches auf einer kontinuierlichen aeroben Kultivierung von Mikroorganismen der Species Alcaligenes eutrophus beruht. Nach den dort gemachten Angaben gelingt es bei diesem Verfahren ein kontrolliertes Wachstum der Mikroorganismenpopulation und gleichzeitig eine zellinterne PHB-Anreicherung dadurch zu erzielen, dass im Gegensatz zum Verfahren der EP-A-15 669 die Versorgung mit den für das Wachstum essentiellen Nähr- und Spurenstoffen, insbesondere die Versorgung mit Stickstoff, von Beginn der Kultivierung an beschränkt wird.

Als Verfahrensvariante ist auch vorgesehen, die Kulturflüssigkeit mit dem Mikroorganismus, der zunächst unter limitierter Nährstoffversorgung bereits auf 25 Gew.-% PHB angereichert wird, in eine zweite Fermentationsstufe zu überführen und dort auf 50 bis 80 Gew.-% PHB anzureichern, wobei jene Nährstoffkomponente, deren Konzentration schon in der ersten Stufe zur Eindämmung des Wachstums beschränkt wurde, in der zweiten Stufe der Nährlösung überhaupt nicht mehr zugesetzt wird, da während der Speicherphase ein Wachstum überhaupt nicht mehr erwünscht ist.

Mit dem Verfahren der EP-A-46 344 wird zwar eine bessere Ausnützung der Kohlenstoffquelle im Nährmedium und eine verbesserte Speicherung der PHB erreicht, es erweist sich aber als nachteilig, dass durch die Unterversorgung mit wachstumsessentiellen Nährstoffen sowohl die spezifische Wachstumsgeschwindigkeit als auch die PHB-Bildungsgeschwindigkeit deutlich herabgesetzt wird.

Ein weiterer Nachteil bei diesem Verfahren ist darin zu sehen, dass bei der Species Alcaligenes eutrophus der optimale Temperaturbereich für die Fermentierung verhältnismässig tief liegt. Der Mikroorganismus muss daher unter starker Kühlung bei 30 bis 34°C in einem Temperaturbereich gezüchtet werden, in dem sowohl Wachstum als auch PHB-Speicherung langsamer verlaufen, als es bei Mikroorganismen mit einer grösseren Thermotoleranz, die bei höherer Temperatur kultiviert werden können, der Fall ist.

Aus den genannten Gründen wird daher bei den bekannten Verfahren die Wirtschaftlichkeit der PHB-Herstellung durch die langen Verweilzeiten und die entsprechend niedrigen Verdünnungraten beim kontinuierlichen Betrieb des Fermenters beeinträchtigt.

Als Kohlenstoffquelle unter den Kohlehydraten dient beim Verfahren der EP-A-46 344 in erster Linie Fructose und nur für den Fall, dass spezielle vom Stamm Alcaligenes eutrophus H 16 abgeleitete Mutanten verwendet werden, kann auf Glucose als Nährstoffquelle ausgewichen werden. Neben dem Umstand, dass die Züchtung und Auslese der Mutanten aus dem Elternstamm arbeitsaufwendig ist, wird durch den Einsatz dieser Mutanten das Angebot an verwertbaren Nährstoffquellen, die eine wirtschaftliche Herstellung von PHB ermöglichen würden, nicht wesentlich erweitert, da die in grosser Menge zur Verfügung stehenden Disaccharide, die als Hauptlieferant für Glucose dienen, beispielsweise die in der Melasse oder industriellen Zuckerlösungen enthaltene Saccharose, auch für die von Alcaligenes eutrophus H 16 abgeleiteten Mutanten nicht verwertbar sind.

Neben Alcaligenes eutrophus werden in der EP-A-46 344 zwar auch noch andere Species der Gattung Alcaligenes als brauchbar für die PHB-Speicherung erwähnt, beispielsweise Alc. faecalis, Alc. ruhlandii, Alc. latus, Alc. aquamarinus, jedoch werden für keine dieser Species nähere Angaben über Züchtungs- und Anreicherungsbedingungen gemacht und die Durchführung der Erfindung ist in der Beschreibung und in den Beispielen nur mit Stämmen von Alcaligenes eutrophus geoffenbart.

Demgegenüber liegt dieser Erfindung die Aufgabe zugrunde, ein wirtschaftlicheres zweistufiges Verfahren zur kontinuierlichen fermentativen Herstellung von PHB mit einem hohen Etragskoeffizienten und einer verbesserten Anreicherung von PHB im Zellmaterial des Mikroorganismus unter Ausnützung von billigeren Kohlenstoffquellen zu schaffen, bei dem die den bekannten Verfahren anhaftenden Nachteile vermieden werden.

Bei der Lösung dieser Aufgabe hat sich nun überraschenderweise gezeigt, dass ein sehr rasches Wachstum der Mikroorganismenpolulation mit gleichzeitiger effektiver PHB-Speicherung bei einer hohen Verdünnungsrate erzielt werden und die Abhängigkeit des Mikroorganismus von der Natur der verwertbaren Kohlenstoffquelle weitgehend beseitigt werden kann, wenn man Stämme von Acaligenes latus oder deren Mutanten im Temperaturbereich von 36 bis 42°C in einem zweistufigen Fermentationsverfahren unter Bedingungen kultiviert, die bisher für die fermentative Herstellung von PHB nicht bekannt waren.

Zu den wesentlichen Merkmalen dieses neuen verbesserten Produktionsverfahrens für PHB gehört, dass man im Gegensatz zum bekannten Stand der Technik durch eine unbeschränkte Nährstoffversorgung, einschliesslich einer vollständigen und ausreichenden Versorgung mit Stickstoff und Phorphor, die Reproduktion und das rasche Wachstum der Mikroorganismenpopulation fördert und überraschenderweise gleichzeitig eine wirksame intrazelluläre PHB-Speicherung erzielt, ohne dass man die Kultur wachstumslimitierenden Bedingungen unterzieht.

Es wurde gefunden, dass unter diesen Bedingungen die PHB-Herstellung in besonders produktiver Weise durchgeführt und eine gute Ausnützung der Kohlenstoffquelle im Nährmedium erreicht werden kann, wenn man den Mikroorganismus kontinuierlich in zwei getrennten, aufeinanderfolgenden Kulturstufen bei unbeschränkter Nährstoffversorgung züchtet, in der zweiten Stufe aber die Züchtung bei einem gegenüber der ersten Stufe geringeren Gelöstsauerstoffgehalt im Nährmedium fortsetzt.

Auf diese Weise entstehen innerhalb von kürzeren Verweilzeiten im Fermenter Biomassen mit einer bedeutend besseren Anreicherung an PHB als es dem Stand der Technik entspricht, wodurch die Herstellung und Isolierung der PHB auf wirtschaftlichere Weise, als es bisher möglich war, gelingt.

Gegenstand der vorliegenden Erfindung ist demnach ein Verfahren zur biotechnologischen Herstellung von Poly-D(−)-3-hydroxybuttersäure durch aerobe Kultivierung eines Mikroorganismus der Gattung Alcaligenes in einem wässerigen Kulturmedium, das Quellen für assimilierbaren Kohlenstoff, Stickstoff und Phosphor, sowie das für das Wachstum des Mikroorganismus erforderliche Angebot an Spurennährstoffen enthält, wobei das Verfahren dadurch gekennzeichnet ist, dass man einen Stamm von Alcaligenes latus oder eine von diesem Mikroorganismus abgeleitete, PHB-speichernde Mutante bei vollständiger und für das Wachstum des Mikroorganismus optimaler Nährstoffversorgung unter unlimitierten Wachstumsbedingungen im Temperaturbereich von 36 bis 42°C in zwei getrennten, aufeinanderfolgenden Fermentierungsstufen unter sterilen Bedingungen kontinuierlich züchtet, indem man die in der ersten Stufe bei einem Gelöstsauerstoffgehalt von 25 bis 50% des Sättigungswerts für Luft gezüchtete, PHB-hältige Mikroorganismenpopulation zusammen mit der Kulturlösung kontinuierlich in eine zweite Fermentationsstufe überführt, in der die Züchtung bei einem Gelöstsauerstoffgehalt von 8 bis 15% des Sättigungswertes für Luft fortgesetzt wird, und aus der dabei erhaltenen Biomasse die PHB auf übliche Weise durch Extraktion gewinnt.

Zur Durchführung des Verfahrens eignen sich alle bekannten Stämme von Alcaligenes latus, beispielsweise die Stämme DSM (Deutsche Sammlung von Mikroorganismen) Nr. 1122, DSM Nr. 1123 oder DSM Nr. 1124.

Die morphologischen Eigenschaften dieser Stämme sind in der Literatur bei Palleroni et al. im Int. Journ. of Systematic Bacteriology $\underline{28}$, 416 - 428, 1978 beschrieben und in Katalogen von Kultursammlungen, beispielsweise in der American Type Culture Collection (ATCC), verzeichnet. Kulturproben der durch DSM-Nummern identifizierten Mikroorganismen sind von der Deutschen Sammlung für Mikroorganismen in Göttingen der Gesellschaft für biotechnologische Forschung, MBH in Stöckheim, Deutsche Bundesrepublik für den Fachmann frei erhältlich.

Stämme von Alcaligenes latus haben die für eine wirtschaftliche PHB-Produktion vorteilhafte Eigenschaft, dass sie in der Lage sind, eine breite Palette von Kohlenstoffquellen zum Wachstum und zur PHB-Speicherung zu verwerten. Als Kohlenstoffquellen sind beispielsweise zu nennen Kohlehydrate, wie D-Glucose, D-Fructose, Lactose, Maltose, Saccharose, Stärke, Melasse, Betain, Grünsirup, Hydrol, Zellulosehydrolysate; organische Säuren bzw. deren Ester und Salze, wie wasserlösliche Salze der Glukon-, 2-Keto-glukon-, Ameisen-, Essig-, Butter-, Isobutter-, L-Malon-, D-(−)-Wein-, Aconit-, Itacon-, m-Hydroxybenzoe-, p-Hydroxybenzoe, Gentisin-, Protocatechu- und Mandelsäure; Alkohole wie n-Propanol, Isopropanol, 2,3-Butylenglycol, Propylenglycol, Glycerin; Aminosäuren, wie β-Alanin, L-Alanin, L-Serin, L-Threonin, L-Leucin, L-Citrullin, L-Ornithin, L-Aminobutyrat, L-Aspartat, L-Asparagin, L-Glutamat, L-Prolin, Hippurat, Sarcosin und Creatin; oder auch Amine, wie Butylamin.

Gemäss ihren Eigenschaften als fakultativ chemolithoautotrophe Mikroorganismen sind die Stämme von Alcaligenes latus aber auch noch in der Lage, neben den bisher aufgezählten Kohlenstoffquellen Kohlendioxid zu verwerten, wenn Kohlendioxid in einem Gemisch mit Wasserstoff und Sauerstoff zusammen als einzige Kohlenstoffquelle angeboten wird.

Da die Wirtschaftlichkeit eines biotechnologischen Verfahrens zur Herstellung von PHB sehr wesentlich vom Preis der Kohlenstoffquelle im Nährsubstrat abhängt, ist die Verwertbarkeit einer so grossen Anzahl von Verbindungen beim erfindungsgemässen Fermentierungsverfahren von grossem Vorteil, da auf die jeweils billigste Kohlenstoffquelle ausgewichen werden kann.

Die ausgezeichnete Verwertbarkeit der leicht zugänglichen Disaccharide ist mit Rücksicht auf die Verfügbarkeit des Nährsubstrats und die Wirtschaftlichkeit der PHB-Herstellung ein überraschendes und vorteilhaftes Merkmal des erfindungsgemässen Verfahrens. In einer bevorzugten Ausführungsform wird dem Mikroorganismus Saccharose, die in industriellen Zuckerlösungen, beispielsweise Grünsirup, oder in Abfallprodukten der Zuckergewinnung, beispielsweise Rübenmelasse, enthalten ist, als Kohlenstoffquelle angeboten, wobei wiederum die Kultivierung des Stammes Alcaligenes latus DSM Nr. 1123 mit Saccharose wegen der hohen PHB-Produktbildungsgeschwindigkeit besonders vorteilhaft ist.

Werden dem Mikroorganismus aus der Gruppe assimilierbarer Kohlenstoffverbindungen solche angeboten, die zugleich Stickstoff im Molekül haben, kann der Kohlenstoff- und Stickstoffbedarf aus derselben Quelle gedeckt werden. Als Stickstoffquelle werden von den Alcaligenes latus-Stämmen auch noch Ammoniak, Ammoniumsalze, beispielsweise Ammoniumchlorid oder Ammoniumsulfat, und Nitrate verwertet. Weiters kann der notwendige Stickstoffbedarf auch durch den Stickstoffgehalt von industriellen und biologischen Abwässern weitgehend gedeckt werden.

Die Zusammensetzung der Nährlösung hinsichtlich ihrer weiteren mineralischen Komponenten umfasst Phosphor, beispielsweise in Form von Natriumhydrogenphosphat oder Kaliumhydrogenphosphat, Magnesium, beispielsweise als Magnesiumsulfat, Calcium beispielsweise als Calciumchlorid und Eisen beispielsweise in Form von Eisen (III)-chlorid, Eisensulfat oder Eisen (III)-Ammoniumcitrat. Weitere Spurenmineralstoffe, die für das Wachstum essentiell sind, können dem Nährmedium bevorzugt in Form einer Spurenlösung zugegeben werden, die beispielsweise folgende Zusammensetzung hat:

| | |
|---|---|
| $ZnSO_4 \cdot 7H_2O$ | 100 mg/l |
| $MnCl_2 \cdot 4H_2O$ | 30 mg/l |
| $H_3BO_3$ | 300 mg/l |
| $CoCl_2 \cdot 6H_2O$ | 200 mg/l |
| $CuSO_3 \cdot 5H_2O$ | 10 mg/l |
| $NiCl_2 \cdot 6H_2O$ | 20 mg/l |
| $NaMoO_4 \cdot 2H_2O$ | 30 mg/l |

Bei der Durchführung des erfindungsgemässen Verfahrens geht man zweckmässigerweise so vor, dass man zunächst eine oder mehrere Vorkulturen, beispielsweise in einem flüssigen Nährmedium, herstellt und dann eine im Produktionsfermenter vorbereitete Nährlösung der angegebenen Zusammensetzung mit der gut angewachsenen Vorkultur im Verhältnis von etwa 1:5 bis 1:15 impft.

Während der gesamten Dauer der Kultivierung wird in beiden Stufen der Fermentierung durch die Ergänzung der verbrauchten Nährsubstrate dafür Sorge getragen, dass die Quellen für Kohlenstoff, Stickstoff und Phosphor, sowie für sämtliche organischen und anorganischen Spurennährstoffe in der für das Bakterienwachstum optimalen Konzentration gehalten werden. Unter optimaler Nährstoffversorgung im Sinne der vorliegenden Erfindung, ist die Züchtung des Mikroorganismus in einem Kulturmedium zu verstehen, das über die gesamte Dauer der Fermentierung hinweg alle Komponenten des vollständigen Nährstoffangebotes in solchen Konzentrationen enthält, dass in der Kultur unlimitierte Wachstumsbedingungen herrschen und der Mikroorganismus keinen Mangel und keine Beschränkung in der Versorgung mit wachstumsessentiellen Nährstoffen, beispielsweise mit Stickstoff oder Phosphor, erfährt.

Die optimale Versorgung mit Stickstoff für das Bakterienwachstum wird nach der Methode von Warburg beschrieben in Manometric Techniques von Umbreit W.W., Burris R.H., Stauffer J.S., Burges Publishing Company, Minneapolis, USA, 1964 am Warburg Respirator bestimmt. Für die Stämme von Alcaligenes latus ist die optimale Stickstoffversorgung, ohne für das Wachstum limitierende Bedingungen zu schaffen, in der Regel dann gegeben, wenn im Kulturmedium während der gesamten Fermentierung eine Konzentration von mindestens 200 mg Ammoniumsulfat pro Liter Kulturflüssigkeit entsprechend einer Stickstoffkonzentration von mindestens 45 mg/l vorhanden ist, wobei in der ersten Fermentationsstufe eine Stickstoffkonzentration von etwa 50 bis 320 mg/l wiederum besonders bevorzugt ist.

Für die optimale Phosphorversorgung ist eine Konzentration von mindestens 500 mg/l Phosphor empfehlenswert, wobei die Einhaltung einer Konzentration von 600 mg bis 1,2 g/l wiederum besonders bevorzugt ist.

Als Kohlenstoffquelle enthält eine für das Verfahren optimale Nährlösung beispielsweise von 1,5 bis 40 g Saccharose pro Liter des Kulturmediums, wobei in der ersten Fermentationsstufe eine Konzentration von 4 bis 10 g/l Saccharose besonders bevorzugt ist. Bei der Kultivierung von Stämmen von Alcaligenes latus unter den erfindungsgemässen Bedingungen tritt überraschenderweise keine ausgeprägte Speicherphase für PHB in Abhängigkeit von der Nährstofflimitierung, beispielsweise durch Unterversorgung mit Stickstoff, auf, sondern es ist eine mit dem Wachstum assoziierte, äusserst effiziente PHB-Anreicherung unter für den Mikroorganismus günstigen Wachstumsbedingungen bei vollständiger Nährstoffversorgung zu beobachten.

Die wachstumsassoziierte PHB-Speicherung bei uneingeschränkter Nährstoffversorgung hat den Vorteil, dass weder die spezifischen Wachstumsgeschwindigkeiten noch die PHB-Produktbildungsgeschwindigkeiten durch Nährstoffmangel herabgesetzt werden und daher eine grössere Konzentration an Biomasse mit einer bessern PHB-Anreicherung innerhalb von wesentlich kürzeren Fermentierzeiten gebildet wird, als es bei den bekannten Verfahren, die unter limitierter Nährstoffversorgung arbeiten, der Fall ist.

Aufgrund dieser hohen Wachstums- und Produktbildungsgeschwindigkeiten werden beim erfindungsgemässen Verfahren in der ersten Fermentierungsstufe bei einer PHB-Speicherung von mehr als 70 Gew.-% des Zelltrockengewichts, beispielsweise Verdünnungsraten D von 0,30 h⁻¹ bis 0,41 h⁻¹ erreicht und in der zweiten Stufe immerhin noch solche von 0,28 h⁻¹ bis 0,33 h⁻¹.

$$D = \frac{F \ \text{Zulauf bzw. Ablauf des Fermenters in Liter pro Stunde}}{V \ \text{Arbeitsvolumen des Fermenters in Litern}}$$

(Die Verdünnungsrate D = ... und hat die Dimension/h⁻¹/).

Die Nährlösung wird in der ersten Fermentierungsstufe zweckmässigerweise in einem kontinuierlichen Zulaufverfahren in den Fermenter, der bevorzugt ein Fermentierkessel ist, eingebracht, bis das maximale Arbeitsvolumen des Fermenters erreicht ist. Nach Erreichen des maximalen Arbeitsvolumens wird das Verfahren kontinuierlich weitergeprüft, indem der Kultur einerseits in konstantem Strom frische Nährlösung zugeführt und andererseits dem Fermenter eine dem Zufluss äquivalente Menge der Nährlösung mit der gebildeten, PHB-hältigen Mikroorganismenpopulation entnommen und in den zweiten Fermenter übergeführt wird.

Die Züchtung erfolgt unter aeroben Bedingungen, beispielsweise unter Sauerstoff- oder Luftzufuhr, wobei zweckmässigerweise der Gelöstsauerstoffgehalt durch Variation der eingetragenen Luft- oder Sauerstoffmenge pro Zeiteinheit in der ersten Stufe in einem Bereich von 25 bis 50% des Sättigungswertes für Luft gehalten wird. Zur Förderung von ungestörtem Wachstum und PHB-Anreicherung ist es vorteilhaft, sterile Luft in das gerührte Kulturmedium einzutragen. Es ist auch möglich, durch Variation der Rührerdrehzahl den Gelöstsauerstoffgehalt im gewünschten Bereich zu halten.

Die Verdünnungsraten werden unter den angegebenen Bedingungen zweckmässigerweise so gewählt, dass die Mikroorganismenpopulation im ersten Fermenter nach Erreichen eines stabilen Gleichgewichtszustandes auf einer Konzentration von etwa 15 bis 25 g bakterieller Trockenmasse pro Liter Kulturflüssigkeit bei einem PHB-Gehalt von mindestens 60 Gew.-% des Zelltrockengewichts gehalten wird, wobei wiederum ein PHB-Gehalt von mindestens 70 Gew.-% für eine ökonomische Durchführung des Verfahrens besonders vorteilhaft ist.

Der zweite Fermenter kann die gleiche Form wie der erste haben und als Rührkessel ausgebildet sein. Da die Verdünnungsrate in der zweiten Fermentierungsstufe in der Regel geringer als in der ersten Stufe ist, muss zur Aufrechterhaltung des kontinuierlichen Betriebs die Züchtung des Mikroorganismus in der zweiten Stufe in einem grösseren Fermenter fortgesetzt werden, dessen Arbeitsvolumen so bemessen ist, dass den unterschiedlichen Verdünnungsraten in der ersten und zweiten Stufe und dem zusätzlichen Nährstoffzulauf Rechnung getragen wird.

Die im Zulauf aus dem ersten Fermenter enthaltene Restkonzentration an Nährstoffen wird durch Zugabe von frischen Nährstoffen laufend so weit ergänzt, dass auch in der zweiten Stufe eine optimale und vollständige Nährstoffversorgung gewährleistet ist. Durch diese Massnahme wird erreicht, dass die Wachstumsphase während der gesamten Dauer der Fermentierung aufrechterhalten wird und die zellinterne PHB-Speicherung wachstumsassoziiert mit hoher Geschwindigkeit erfolgt. Ausserdem ist unter Wachstumsbedingungen überraschenderweise eine relative Vergrösserung der speichernden Zellen in der zweiten Fermentierungsstufe zu beobachten, so dass die Abtrennung der Biomasse aus der Nährlösung einfacher durchzuführen ist. Dadurch können die Zellmassen beispielsweise durch einfaches Filtrieren gut abgetrennt werden und man kann sich aufwendigere Einrichtungen, beispielsweise die Anschaffung von Zentrifugen für den Trennvorgang, ersparen.

Da in der zweiten Fermentationsstufe der Zeitpunkt des grössten expotentiellen Wachstums der Mikroorganismenpopulation bereits überschritten ist, kann es vorteilhaft sein, die Nährstoffkonzentration an der unteren Grenze der oben für die optimale Nährstoffversorgung angegebenen Bereiche zu halten, um eine möglichst gute Nutzung der eingesetzten Nährstoffe zu erzielen.

Die Zusammensetzung des Nährmediums und der Zulauf der frischen Nährstoffe werden in der zweiten Fermentierungsstufe daher vorteilhafterweise so geregelt, dass die Konzentration der Kohlenstoffquelle in der Kulturflüssigkeit den Wert von etwa 1,5 g Saccharose pro Liter Kulturflüssigkeit nicht unterschreitet, wobei eine Konzentration von 1,5 bis 2,5 g wiederum besonders bevorzugt ist. Die Stickstoffkonzentration beträgt mindestens 45 mg, bevorzugt aber etwa von 50 bis 60 mg pro Liter Kulturflüssigkeit.

Der Gelöstsauerstoffgehalt der Nährlösung wird in der zweiten Fermentierungsstufe auf 8 bis 15%, bevorzugt auf 8 bis 10% des Sättigungswertes für Luft abgesenkt. Die Absenkung des Gelöstsauerstoffgehalts hat den Vorteil, dass einerseits die Kosten für den Sauerstofftransfer verringert werden und gleichzeitig die Kohlenstoffquelle im Nährmedium besser ausgenützt werden kann. Die bessere Ausnützung der Nährstoffquelle ist vor allem deshalb von Bedeutung, weil dadurch die Restkonzentration an Kohlenstoff im Ablauf nach Ende der Fermentierung wesentlich verringert werden kann.

Unter den Bedingungen des erfindungsgemässen Verfahrens ist der Mikroorganismus imstande, den PHB-Gehalt in den Zellen bis zu etwa 84 Gew.-% des Zelltrockengewichts anzureichern. Bei der praktischen Durchführung des Verfahrens wird in der zweiten Fermentierungsstufe die Verdünnungsrate zweckmässigerweise so eingestellt, dass die Mikroorganismenpopulation auf eine Konzentration von 15 bis 35 g Zelltrockengewicht pro Liter Kulturflüssigkeit bei einem PHB-Gehalt von 70 bis 80 Gew.-%, vorzugsweise von 75 bis 80 Gew.-% des Zelltrockengewichts angereichert wird.

Die Stämme von Alcaligenes latus sind im Gegensatz zur Species Alcaligenes eutrophus überraschenderweise thermotoleranter. Diese erhöhte Thermotoleranz zeigt sich darin, dass der optimale Temperaturbereich für die Fermentierung in beiden Stufen bei 36 bis 42°C liegt, wobei wiederum eine Temperatur von 37 bis 39°C bevorzugt ist. Die Züchtung in diesem Temperaturbereich hat neben der beschleunigenden Wirkung auf Wachstum und PHB-Speicherung den Vorteil, dass die Kühlkosten während der Fermentierung erheblich gesenkt werden können. Die Einsparung an Kühlkosten durch die Züchtung bei höherer Temperatur in Verbindung mit kürzeren Verweilzeiten fällt besonders ins Gewicht, da die Kühlkosten in der Regel die Hälfte der Energiekosten betragen, die beim Betrieb eines Fermenters anfallen.

Der pH-Wert der Nährlösung wird während der gesamten Dauer der Fermentation durch laufende Zugabe von Pufferlösung, beispielsweise von Phosphatpufferlösung oder von wässeriger Base, beispielsweise Kalium- oder Natriumhydroxydlösung vorteilhafterweise auf Werte zwischen 6,5 und 7,5, bevorzugt Werte von 6,8 bis 7,2 eingestellt.

Unter den angegebenen Bedingungen kann der kontinuierliche Betrieb des Verfahrens über Wochen aufrechterhalten werden, ohne dass sich in der Zusammensetzung der erhaltenen Biomasse oder beim Ertragskoeffizienten Änderungen ergeben.

Bei der Verwendung von Kohlehydraten als Kohlenstoffquelle werden beim erfindungsgemässen Verfahren Ertragskoeffizienten $Y_{x/s} = 0,42$ bis $0,46$ in beiden Verfahrensstufen erzielt, d.h. pro Gramm des eingesetzten Kohlehydrats werden 0,42 bis 0,46 g Zelltrockengewicht in Form von PHB-hältiger Biomasse gewonnen.

Im Ausmasse des Zulaufs in den zweiten Fermenter wird Kulturlösung mit der an PHB angereicherten Mikroorganismenpopulation entnommen und die Zellmassen durch übliche Trennmethoden, wie Dekantieren oder Zentrifugieren, bevorzugt aber durch Filtrieren abgetrennt und daraus die PHB auf übliche Weise durch Extrahieren gewonnen, beispielsweise nach dem Verfahren der US-PS 4 101 533.

Die von der Biomasse befreite Nährlösung enthält in der Regel nur mehr geringe Nährstoffkonzentrationen.

Zur Ausnützung der zurückbleibenden Nährstoffe kann die von Zellen befreite Nährlösung im Kreislauf in den ersten Fermenter zurückgeführt, zur Erreichung der optimalen Nährstoffkonzentration mit frischer Nährlösung versetzt und für weiteren Züchtungen verwendet werden.

Im folgenden Beispiel ist die Erfindung näher erläutert.:

*Beispiel*

In einem sterilen Fermenter mit einem Arbeitsvolumen von 15 Litern werden 10 Liter eines Nährmediums I mit einem Gehalt von 1,5 g/l $(NH_4)_2SO_4$ und 15 g/l Saccharose steril filtriert vorgelegt und mit 1 l einer gut angewachsenen Vorkultur von Alcaligenes latus DSM Nr. 1123 beimpft.

Das Medium I hat folgende Zusammensetzung:

| | | |
|---|---|---|
| $Na_2HPO_4 \cdot 2H_2O$ | : | 4,5 g/l |
| $KH_2PO_4$ | : | 1,5 g/l |
| $MgSO_4 \cdot 7H_2O$ | : | 0,2 g/l |
| $CaCl_2 \cdot 2H_2O$ | : | 0,02 g/l |
| Spurenlösung | : | 2 ml/l |
| Fe III-$NH_4$-Citrat | : | 0,05 g/l |

Die Spurenlösung hat folgende Zusammensetzung:

| | | |
|---|---|---|
| $ZuSO_4 \cdot 7H_2O$ | : | 100 mg/l |
| $MnCl_2 \cdot 4H_2O$ | : | 30 mg/l |
| $H_3BO_3$ | : | 300 mg/l |
| $CoCl_2 \cdot 6H_2O$ | : | 200 mg/l |
| $CuCO_4 \cdot 5H_2O$ | : | 10 mg/l |
| $NiCl_2 \cdot 6H_2O$ | : | 20 mg/l |
| $NaMoO_4 \cdot 2H_2O$ | : | 30 mg/l |

Der Gelöstsauerstoffgehalt wird durch Variation der Rührerdrehzahl und Belüftungsrate im Bereich zwischen 28 und 30% des Sättigungswertes für Luft gehalten, die Arbeitstemperatur beträgt 37°C. Während der Fermentation wird der pH-Wert der Nährlösung durch automatische Titration mit einer 10%-igen sterilen wässerigen NaOH-Lösung bei 7,0 konstant gehalten.

Im weiteren Verlauf der Züchtung werden die Nährsubstrate mittels eines kontinuierlichen Zulaufverfahrens durch einen den Verbrauchsgeschwindigkeiten und der Verdünnung angepassten Zulauf mit frischen Nährlösungen konstant gehalten. Die frischen Nährlösungen bestehen aus Saccharoselösung mit einer Konzentration von 300 g/l, $(NH_4)_2SO_4$-Lösung mit einer Konzentration von 200 g/l und frischem Medium I.

Wenn das maximale Arbeitsvolumen des Reaktors erreicht ist, wird eine kontinuierliche Arbeitsweise in der Form eingerichtet, dass der Kultur einerseits in konstantem Strom frische Nährlösung zugeführt wird, andererseits eine dem Zufluss äquivalente Menge an Nährlösung entnommen und im kontinuierlichen Strom in einen zweiten Fermenter mit einem Arbeitsvolumen von 25 l überführt wird. Die frisch zugeführte Nährlösung enthält Saccharose in einer Konzentration von 40 g/l, $(NH_4)_2SO_4$ in einer Konzentration von 4,6 g/l und frisches Medium I in der oben angegebenen Zusammensetzung. Auf diese Weise wird nach einiger Zeit ein stabiler Gleichgewichtszustand erreicht, wobei die Kulturbrühe in der ersten Fermentationsstufe 16,5 g/l trockener Biomasse mit einem PHB-Gehalt von 71 Gew.-% enthält.

Der Überlauf zum zweiten Fermenter enthält noch eine Restkonzentration an $(NH_4)_2SO_4$ von 0,45 g/l und eine Restkonzentration an Saccharose von etwa 4,1 g/l. Im Gleichgewichtszustand werden 6,0 g/l an frischem Nährmedium zugeführt, was einer Verdünnungsrate von $D = 0,4$ h$^{-1}$ entspricht.

Im zweiten Fermenter, der ein maximales Arbeitsvolumen von 25 l aufweist, werden Temperatur und pH-Werte wie in der ersten Fermentationsstufe eingestellt und konstant gehalten. Dagegen wird durch Variation von Belüftungsrate und Rührerdrehzahl die Gelöstsauerstoffkonzentration auf 8 bis 10% des Sättigungswertes für Luft herabgesetzt. Zusätzlich zum Zulauf aus der ersten Fermentationsstufe wird der zweite Fermenter noch mit 1,5 l pro Stunde einer sterilen wässerigen Nährlösung, die 26,64 g/l Ammonsulfat und Saccharose mit einer Konzentration von 236 g/l enthält, versorgt um die Konzentration dieser beiden Substrate auch in dieser Phase nicht limitierend werden zu lassen. Durch diese Massnahmen wird die Wachstumsphase während der gesamten Züchtung aufrechterhalten.

Unter diesen Bedingungen erhält man im Auslauf der zweiten Fermentationsstufe eine Kulturbrühe, die 35,5 g pro Liter trockener Biomasse mit einem konstanten PHB-Anteil von 79 Gew.-% enthält. In dieser zweiten Stufe ergibt sich eine Verdünnungsrate von D = 0,30 h$^{-1}$, was einem Gesamtdurchfluss von 7,5 l pro Stunde entspricht.

Diese kontinuierliche Fermentierung kann über einen Zeitraum von drei Wochen problemlos aufrechterhalten werden, ohne dass sich hinsichtlich der Etragskoeffizienten $Y_{x/s}$ = 0,46 oder an der Zusammensetzung der Biomasse Änderungen ergeben.

In der nachfolgenden Tabelle I sind die Betriebsbedingungen bei der kontinuierlichen zweistufigen Herstellung von PHB mit Alcaligenes latus Stamm DSM Nr. 1123 zusammengestellt.

### TABELLE I

| | Stufe 1 | Stufe 2 |
|---|---|---|
| Arbeitsvolumen | 15 l | 25 l |
| Rührung | Querblatt | Querblatt |
| Temperatur | 37°C | 37°C |
| pH-Wert | 7,0 | 7,0 |
| O$_2$-Konzentration (% der Luftsättigung) | 28-30% | 8-10% |
| Verdünnungsrate D | 0,4 h$^{-1}$ | 0,3 h$^{-1}$ |
| Kohlenstoffquelle | Saccharose | Saccharose |
| Saccharosekonzentration im Zulauf | 40 g/l | --- |
| Stickstoffquelle | (NH$_4$)$_2$SO$_4$ | (NH$_4$)$_2$SO$_4$ |
| Konzentration (NH$_4$)$_2$SO$_4$ Zulauf | 4,6 g/l | --- |
| Restkonzentration Saccharose | 4,1 g/l | 2,1 g/l |
| Restkonzentration (NH$_4$)$_2$SO$_4$ | 0,45 g/l | 0,085 g/l |
| Saccharoseverbrauch | 35,9 g/l | --- |
| (NH$_4$)$_2$SO$_4$-Verbrauch | 4,15 g/l | --- |
| Biomassekonzentration | 16,5 g/l | 35,5 g/l |
| PHB-Gehalt der Biomasse | 71% d.ZTG | 79% |
| Produktivität Biomasse | 99 g/h | 266,4 g/h |
| Produktivität PHB | 70,4 g/h | 210,46 g/h |
| $Y_x$,Saccharose | 0,46 g/g | 0,46 g/g |
| $Y_x$,(NH$_4$)$_2$SO$_4$ | 3,98 g/g | 3,98 g/g |
| (NH$_4$)$_2$SO$_4$-Konzentration im Teilstromfermenter 2 | --- | 26,64 g/l (1,5 l/h) |
| Saccharose-Konzentration im Teilstromfermenter 2 | --- | 236 g/l (1,5 l/h) |
| Biomasse-Konzentration im Zulauf | | 13,2 g/l |

## Patentansprüche

1. Verfahren zur Herstellung von Poly-D(–)-3-hydroxybuttersäure durch aerobe Kultivierung eines Mikroorganismus der Gattung Alcaligenes in einem wässerigen Kulturmedium, das Quellen für assimilierbaren Kohlenstoff, Stickstoff und Phosphor, sowie das für das Wachstum des Mikroorganismus erforderliche Angebot an Spurennährstoffen enthält, dadurch gekennzeichnet, dass man einen Stamm von Alcaligenes latus bei vollständiger und für das Wachstum des Mikroorganismus optimaler Nährstoffversorgung unter unlimitierten Wachstumsbedingungen im Temperaturbereich von 36°C bis 42°C in zwei getrennten und aufeinanderfolgenden Fermentierungsstufen unter sterilen Bedingungen kontinuierlich züchtet, indem man die in der ersten Stufe bei einem Gelöstsauerstoffgehalt von 25 bis 50% des Sättigungswertes für Luft gezüchtete, PHB-hältige Mikroorganismenpopulation zusammen mit der Kuturlösung kontinuierlich in eine zweite Fermentierungsstufe überführt, in der die Züchtung bei einem Gelöstsauerstoffgehalt von 8 bis 15% des Sättigungswertes für Luft fortgesetzt wird, und aus der dabei erhaltenen Biomasse die PHB auf übliche Weise durch Extraktion gewinnt.

2. Verfahren nach Anspruch 1, dadurch gekennzeichnet, dass die Stämme von Alcaligenes latus DSM Nr. 1122, DSM Nr. 1123 oder DSM Nr. 1124 eingesetzt werden.

3. Verfahren nach den Ansprüchen 1 und 2, dadurch gekennzeichnet, dass man als Kohlenstoffquelle Saccharose verwendet.

4. Verfahren nach den Ansprüchen 1 bis 3, dadurch gekennzeichnet, dass man den Stamm Alcaligenes latus DSM Nr. 1123 mit Saccharose als einziger Kohlenstoffquelle züchtet.

5. Verfahren nach den Ansprüchen 1 bis 4, dadurch gekennzeichnet, dass man die Züchtung in einem Nährmedium vornimmt, welches pro Liter Kulturflüssigkeit 1,5 bis 40 g Saccharose als Kohlenstoffquelle enthält.

6. Verfahren nach den Ansprüchen 1 bis 5, dadurch gekennzeichnet, dass man die Züchtung in einem Nährmedium vornimmt, welches während der gesamten Dauer der Fermentierung pro Liter Kulturflüssigkeit mindestens 45 mg Stickstoff enthält.

7. Verfahren nach den Ansprüchen 1 bis 6, dadurch gekennzeichnet, dass man die Züchtung in einem Nährmedium vornimmt, welches pro Liter Kulturflüssigkeit in der ersten Fermentationsstufe von 4 bis 10 g Saccharose als Kohlenstoffquelle und von 50 bis 320 mg Stickstoff und in der zweiten Fermentationsstufe von 1,5 bis 2,5 g Saccharose als Kohlenstoffquelle und von 50 bis 60 mg Stickstoff enthält.

8. Verfahren nach den Ansprüchen 1 bis 7, dadurch gekennzeichnet, dass man die Züchtung in einem Nährmedium vornimmt, welches pro Liter Kulturflüssigkeit mindestens 500 mg Phosphor enthält.

9. Verfahren nach den Ansprüchen 1 bis 8, dadurch gekennzeichnet, dass man in der ersten Fermentationsstufe eine Mikroorganismenpopulation mit einer Konzentration von 15 bis 25 g bakterieller Trockenmasse pro Liter Kulturflüssigkeit bei einem PHB-Gehalt von mindestens 70 Gew.-% züchtet.

10. Verfahren nach den Ansprüchen 1 bis 9, dadurch gekennzeichnet, dass man die Züchtung in der zweiten Fermentationsstufe bei einem Gelöstsauerstoffgehalt von 8 bis 10% des Sättigungswerts für Luft durchführt.

11. Verfahren nach den Ansprüchen 1 bis 10, dadurch gekennzeichnet, dass man in der zweiten Fermentationsstufe den PHB-Gehalt der Biomasse auf 75 bis 80 Gew.-% des Zelltrockengewichts anreichert.

12. Verfahren nach den Ansprüchen 1 bis 11, dadurch gekennzeichnet, dass man die Züchtung bei 37 bis 39°C durchführt.

13. Verfahren nach den Ansprüchen 1 bis 12, dadurch gekennzeichnet, dass man die Züchtung bei einem pH-Wert vn 6,8 bis 7,2 durchführt.

## Claims

1. Process for the preparation of poly-D(-)-3-hydroxybutyric acid by aerobic cultivation of a microorganism of the genus Alcaligenes in an aqueous culture medium which contains sources of assimilable carbon, nitrogen and phosphorus, as well as the amount of trace nutrients necessary for growth of the microorganism, characterized in that a strain of Alcaligenes latus is continuously cultured with a supply of nutrients which is complete and optimal for the growth of the microorganism, under unlimited growth conditions, in the temperature range 36°C to 42°C, in two separate and successive fermentation steps under sterile conditions, in such a way that the microorganism population which has been cultured in the first step at a dissolved oxygen content of 25 to 50% of the saturation value for air and contains PHB is transferred, together with the culture solution, continuously into a second fermentation step in which the culturing is continued at a dissolved oxygen content of 8 to 15% of the saturation value for air, and the PHB is isolated in a customary manner by extraction from the biomass obtained thereby.

2. Process according to Claim 1, characterized in that the strains DSM No. 1122, DSM No. 1123 or DSM No. 1124 of Alcaligenes latus are used.

3. Process according to Claims 1 and 2, characterized in that sucrose is used as carbon source.

4. Process according to Claims 1 to 3, characterized in that the strain Alcaligenes latus DSM No. 1123 is cultured with sucrose as sole carbon source.

5. Process according to Claims 1 to 4, characterized in that the culturing is carried out in a nutrient medium which contains 1,5 to 40 g of sucrose as carbon source per litre of culture liquid.

6. Process according to Claims 1 to 5, characterized in that the culturing is carried out in a nutrient medium which contains at least 45 mg of nitrogen per litre of culture liquid throughout the duration of the fermentation.

7. Process according to Claims 1 to 6, characterized in that the culturing is carried out in a nutrient medium which contains, per litre of culture liquid, from 4 to 10 g of sucrose as carbon source and from 50 to 320 mg of nitrogen in the first fermentation step and from 1.5 to 2.5 g of sucrose as carbon source and from 50 to 60 mg of nitrogen in the second fermentation step.

8. Process according to Claims 1 to 7, characterized in that the culturing is carried out in a nutrient medium which contains at least 500 mg of phosphorus per litre of culture liquid.

9. Process according to Claims 1 to 8, characterized in that, in the first fermentation step, a microorganism population with a concentration of 15 to 25 g of bacterial dry mass per litre of culture liquid is cultured at a PHB content of at least 70% by weight.

10. Process according to Claims 1 to 9, characterized in that the culturing in the second fermentation step is carried out at a dissolved oxygen content of 8 to 10% of the saturation value for air.

11. Process according to Claims 1 to 10, characterized in that the PHB content of the biomass is enriched in the second fermentation step to 75 to 80% by weight of the dry weight of cells.

12. Process according to Claims 1 to 11, characterized in that the culturing is carried out at 37°C to 39°C.

13. Process according to Claims 1 to 12, characterized in that the culturing is carried out at a pH of 6.8 to 7.2.

## Revendications

1. Procédé de préparation d'acide poly-D(-)-3-hydroxybutyrique par culture aérobie d'un micro-organisme du genre Alcaligenes dans un milieu de culture aqueux qui contient des sources de carbone, azote et phosphore assimilables, ainsi qu'un apport en substances nutritives à l'état de traces, nécessaire pour la croissance du micro-organisme caractérisé en ce qu'on cultive en continu, dans des conditions stériles, une souche d'Alcaligenes latus en présence d'une alimentation en substance nutritive complète et optimale pour la croissance du micro-organisme, dans des conditions de croissance non limitées et dans l'intervalle de températures de 36°C à 42°C, dans deux stades de fermentation séparés et successifs, en soumettant la population de micro-organismes contenant le PHB, cultivée dans le premier stade en présence d'un teneur en oxygène dissous de 25 à 50% de la valeur de saturation pour l'air, à un transfert en continu, conjointement avec la solution de culture, dans un deuxième stade de fermentation dans lequel on poursuit l'opération de culture en présence d'une teneur en oxygène dissus de 8 à 15% de la valeur de saturation pour l'air et en récupérant le PHB de manière courante par extraction à partir de la biomasse ainsi obtenue.

2. Procédé selon la revendication 1, caractérisé en ce qu'on introduit les souches d'Alcaligenes latus, DSM N° 1122, DSM N° 1123 ou DSM N° 1124.

3. Procédé selon les revendications 1 et 2, caractérisé en ce qu'on utilise comme source de carbone, le saccharose.

4. Procédé selon les revendications 1 à 3, caractérisé en ce qu'on cultive la souche Alcaligenes latus DSM N° 1123 avec le saccharose comme seule source de carbone.

5. Procédé selon les revendications 1 à 4, caractérisé en ce que l'on procède à la culture dans un milieu nutritif qui contient par litre de liquide de culture, 1,5 à 40 g de saccharose comme source de carbone.

6. Procédé selon les revendications 1 à 5, caractérisé en ce que l'on entreprend la culture dans un milieu nutritif qui contient pendant toute la durée de la fermentation par litre de liquide de culture, au moins 45 mg d'azote.

7. Procédé selon les revendications 1 à 6, caractérisé en ce que l'on entreprend la culture dans un milieu nutritif qui contient par litre de liquide de culture, dans le premier stade de fermentation de 4 à 10 g de saccharose en tant que source de carbone et de 50 à 320 mg d'azote, et dans le deuxième stade de fermentation de 1,5 à 2,5 g de saccharose en tant que source de carbone et de 50 à 60 mg d'azote.

8. Procédé selon les revendications 1 à 7, caractérisé en ce que l'on entreprend la culture dans un milieu nutritif qui contient par litre de liquide de culture, au moins 500 mg de phosphore.

9. Procédé selon les revendications 1 à 8, caractérisé en ce que dans le premier stade de fermentation, on cultive une population de micro-organismes ayant une concentration de 15 à 25 g de masse bactérienne sèche par litre de liquide de culture à teneur en PHB d'au moins 70% en poids.

10. Procédé selon les revendications 1 à 9, caractérisé en ce que l'on effectue la culture dans le deuxième stade de fermentation en présence d'une teneur en oxygène dissous de 8 à 10% de la valeur de saturation pour l'air.

11. Procédé selon les revendications 1 à 10, caractérisé en ce que dans le deuxième stade de fermentation, on enrichit la teneur en PHB de la biomasse à 75 à 80% en poids par rapport au poids sec des cellules.

12. Procédé selon les revendications 1 à 11, caractérisé en ce que l'on effectue la culture à 37°C à 39°C.

13. Procédé selon les revendications 1 à 12, caractérisé en ce que l'on effectue la culture à une valeur de pH de 6,8 à 7,2.